# EUROPEAN PATENT APPLICATION

(11) **EP 1 604 642 A1**
(43) Date of publication of application: **14.12.2005**
(21) Application number: 05252785.0
(22) Date of filing: 06.05.2005
(51) Int. Cl.: A61K 7/32, A45D 40/04

(54) **Antiperspirant composition comprising polyol active, antiperspirant article containing same and method for using same**

(30) Priority: 10.06.2004 US 865007
(71) Applicant: INTERNATIONAL FLAVORS & FRAGRANCES INC., New York New York 10019 (US)
(72) Inventor: Boden, Richard M., Ocean, New Jersey 07712 (US); Lindauer, Jerome I., Hillsdale, New Jersey 07642 (US); Prol, Melanie Beth, Brick, New Jersey 08724 (US)
(74) Representative: Lawrence, John

(57) **Abstract**

Described is a method for inhibiting apocrine gland sweating or eccrine gland sweating comprising the step of topically applying an organic polyol antiperspirant active-containing composition comprising a sweat secretion inhibiting concentration of a C₃-C₆ alkane polyol antiperspirant active which polyol has bonded thereto from three to six hydroxyl moieties and/or monoester, monoether, monohemiacetal, monoacetal, monohemiketal or monoketal derivatives thereof and is selected from the group consisting of glycerine, sorbitol, sorbitol internal cyclic ether anyhydride, mannitol, mannitol internal cyclic ether anhydride, glucitol, glucitol internal cyclic ether anhydride and 1,2,6-hexane triol. Also described are specific organic polyol antiperspirant active-containing compositions useful for carrying out such processes and antiperspirant stick articles.

## Description

### FIELD OF THE INVENTION

Use as an antiperspirant of at least one organic C₃-C₆ alkane polyol antiperspirant active which organic polyol has bonded thereto from three to six hydroxyl moieties and/or one or more monoester, monoether, monohemiacetal, monoacetal, monohemiketal or monoketal derivatives thereof in the absence of any polyvalent metal compounds or hydrocarbyl glycols of the formula R(OH)₂ where R is hydrocarbyl.

### BACKGROUND OF THE INVENTION

Antiperspirant compositions effecting diminution of sweat secreted by the apocrine sweat glands and/or the eccrine sweat glands over a substantial period of time in the form of aqueous creams, lotions, sticks, roll-ons and the like are well known in the art. Antiperspirant formulations have in the past relied on the use of antiperspirant actives which are polyvalent metal compounds, for example, alum and inorganic and organic salts of aluminum, zirconium, zinc, calcium, magnesium, lanthanum and hafnium, such as, aluminum chlorhydroxide, zirconyl hydroxychloride or mixtures of glycine, zirconyl hydroxychloride and aluminum chlorhydroxide as described in U.S. Patents 3,792,068, 5,534,246, and 6,524,562 and mixtures thereof with calcium and/or magnesium salts as described in U.S. Patents 3,998,788 and 6,042,816. The presence of such polyvalent metal-containing compounds in compositions which come into contact with the mammalian epidermal tissue causes adverse physiological reactions to the mammalian epidermal tissue and restricts the type of fragrance formulation which can be used in conjunction with such compositions as a result of the necessary pH constraints on the compositions. Considerable efforts have been made to (i) effect diminution of the effects, including adverse physiological effects of such polyvalent metal compounds, for example, by means of incorporation of the polyvalent metal compound with a polyol as disclosed in U.S. Patent Application 2003/0157044 or with a fatty acid or triglyceride thereof as disclosed in U.S. Patent 6,713,051 or with a 1,2-hexanediol carrier as disclosed in U.S. Patent 5,968,489 or with a glycol-containing base gel as disclosed in U.S. Patent 5,730,963 or in a structured continuous oil phase as disclosed in U.S. Patent 6,737,048 or (ii) to entirely replace such polyvalent metal compound-containing antiperspirant actives with materials which have antiperspirant efficacies similar to those of the typically-employed polyvalent metal-containing compounds, but do not give rise to the adverse physiological reactions which apparently result from the use of antiperspirant compositions containing the polyvalent metal-containing antiperspirant actives. Table I is indicative of attempts to create antiperspirant compositions where the polyvalent metal compound antiperspirant active is attempted to be replaced with an alternative antiperspirant active:

**TABLE I**

| | |
|---|---|
| U.S.Patent or Published Patent Application | Antiperspirant Active |
| U.S.Patent 5,688,495 | acetyl salicylic acid, cetyl alcohol and stearic acid mixture |
| U.S. Patent 5,730,964 | 5-α-reductase inhibitor |
| U.S, Patent 6,001,341 | alkyl ester |
| U.S. Patent Application 2002/0131947 | polyhydric alcohol-modified silicone |
| U.S. Patent Application 2003/0206973 | olive oil, almond oil or wheat germ oil in combination with *Salvia Officinalis* or *Cupressus Semperiens.* |
| U.S. Patent Application 2004/0096409 A1 | copolymer of acyloyl dimethyltaurine and Si- or F-containing component |

The efficacy of the attempted prior art replacement materials for the polyvalent metal compound antiperspirant actives is substantially less than the efficacy of such polyvalent metal compound antiperspirant actives.

Commencing with the application of the earlier findings ((i)as published in the "Alchemist" , American Chemical Society online publication, Zheng and Bollag, December 8, 2003, "Avoiding Thick Skin"; (ii) as published in Hattori and Kaufer, J. Neurochem.1985 Nov.;45(5):1578-84 and abstracted in MEDLINE 4045465, (Title: "Synaptosomal phospholipase D potential role in providing choline for acetylcholine synthesis"); (iii) as published in BMC Neurosci.2001;2(1):16.Epub 2001 Oct 19 and abstracted in MEDLINE 11734063 (Title: "Generation of choline for acetylcholine synthesis by phospholipase informs"); and (iv) as published in Sato et al. Am.J. Physiol. 1979 September;237(3):C177-84 and abstracted in MEDLINE 38671 (Title: "Mechanical properties and functions of the myoepithelium in the eccrine sweat gland")) that (a) phospholipase D signals the eccrine and apocrine sweat glands into action, and (b) glycerin, through its action with phosphlipase D effects moisturization of the mammalian epidermis by means of signaling a repair mechanism for the mammalian epidermal cells, we have found that the action of phospholipase D on surface skin secretions where synaptosomal phospholipase D catalyzes the release of choline from phosphatidylcholine for acetylcholine formation can be substantially and efficaciously inhibited and thus aprocrine gland sweating and eccrine gland sweating is efficaciously inhibited by means of topical application to the epidermal area of said apocrine gland or eccrine gland of one or more organic C₃-C₆ alkane polyols, each of which polyol has bonded thereto from three to six hydroxyl moieties and/or monoester, monoether, monohemiacetal, monoacetal, monohemiketal or monoketal derivatives thereof, e.g., glycerine, used as an antiperspirant active, in the absence of any (i) polyvalent metal compounds, e.g., zirconium, zinc, manganese, calcium, bismuth, lanthanum, copper, hafnium, magnesium or aluminum salts, hydroxides or amine complexes or (ii) hydrocarbyl glycols of the formula R(OH)₂ where R is hydrocarbyl.

The use of such polyvalent metal compound-free and hydrocarbyl glycol-free polyols as antiperspirant inactives is unexpected and unobvious, notwithstanding the disclosures of (a) a process of suppressing odors employing deodorants containing dihydroxycarboxylic acid esters having 2-4 carbon atoms in U.S. Patent 4,005,189 or (b) deodorant compositions consisting essentially of a mixture diglycerol, tripropylene glycol and propylene glycol in U.S. Patent 6,652,842 or (c) the preparation of a "Antiperspirant/Deodorant Base" in Example III (i), at Col. 9, lines 12-24 of U.S. Patent 6,368,633. The chemical nature of the human proteins secreted is disclosed in U.S. Patent Application 2003/0027999. Examples of phospholipid derivatives involved are set forth in PCT Patent Application WO 2004/000854. The fundamentals concerning (i) employment of phospholipids in the human cytoplasmic membrane and (ii) malodour development from sweat secreted from the apocrine gland is set forth at pages 28, 29, 388 and 389 of Brock et al., "Biology of Microorganisms", 6^{th} Ed., 1991, published by Prentice Hall Publishing Co.

### SUMMARY OF THE INVENTION

Our invention is directed to a method for inhibiting apocrine gland sweating or eccrine gland sweating apparently enabled by the action of phospholipase D on surface skin cell secretions whereby synaptosomal phospholipase D catalyzes the release of choline from phosphatidylcholine for acetylcholine formation. The method of our invention specifically involves topically applying to at least one area of occurrence of (i) epidermal apocrine gland sweat secretions or (ii) epidermal eccrine gland secretions, in a phospholipase D inhibiting amount and at a phospholipase D inhibiting rate an organic polyol antiperspirant active-containing composition which is hydrocarbyl diol-free and polyvalent metal compound-free, comprising a phospholipase D inhibiting concentration of a C₃-C₆ alkane polyol antiperspirant active which polyol has bonded thereto from three to six hydroxyl moieties and/or monoester, monoether, monohemiacetal, monoacetal, monohemiketal or monoketal derivatives thereof. More specifically the C₃-C₆ alkane polyol antiperspirant active substances useful in the practice of our invention are one or more of glycerine, sorbitol, sorbitol internal cyclic ether anyhydride, mannitol, mannitol internal cyclic ether anhydride having the structure: 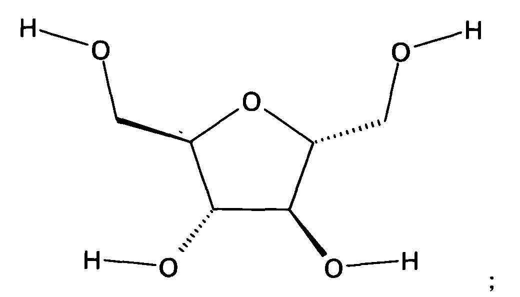 glucitol, glucitol internal cyclic ether anhydride having the structure: 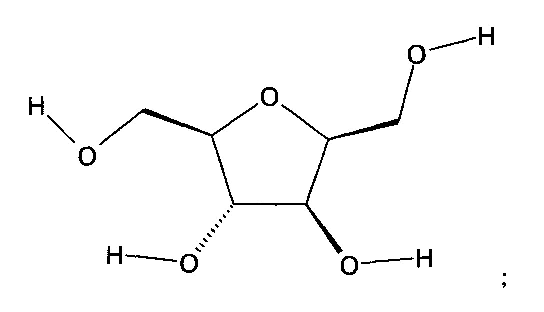 and 1,2,6-hexane triol and/or a C₂-C₁₈ alkyl or alkenyl mono-ester thereof, a C₂-C₁₂ alkyl monoether thereof, a mono[(C₂-C₃ oxy)₁₋₁₅]ether thereof, a mono-C₂-C₈ hemiacetal thereof, a mono-C₁-C₈ acetal thereof, a mono-C₃-C₈ hemiketal thereof and/or a mono-C₃-C₈ ketal thereof.

The terms: "in the absence of any polyvalent metal compounds" and "polyvalent metal compound-free" are herein intended to mean "in the absence of any compounds containing metals having a valence of greater than one including, but not limited to organic and inorganic ionic and non-ionic compounds containing calcium, magnesium, aluminum, zirconium, copper, zinc, bismuth, manganese, germanium, lanthanum or hafnium, such as, aluminum chlorhydroxide, zirconyl hydroxychloride and mixtures of glycine, zirconyl hydroxychloride and aluminum chlorhydroxide". However, the term "polyvalent metal" is not herein intended to include silicon-, sulfur- or carbon-containing compounds.

The terms, "in the absence of glycols of the formula R(OH)₂ where R is hydrocarbyl" and "hydrocarbyl glycol-free" are herein intended to mean "in the absence of such hydrocarbyl diols as 1,2-ethylene glycol; 1,2-propylene glycol; 1,3-propylene glycol; 1,2-hexylene glycol; 2-butene-1,4-diol and the like" wherein the term, "hydrocarbyl" defines a moiety consisting solely of carbon and hydrogen.

Topical application to the area of occurrence of (i) epidermal apocrine gland sweat secretions or (ii) epidermal eccrine gland secretions of organic polyol antiperspirant active-containing composition in accordance with the practice of our invention may be by means of the use of any application device using product forms familiar to those having ordinary skill in the art including, but not limited to solid or gel solid sticks, soft solids or creams, pastes, lotions or other liquids including "roll-on's" and aerosol or pump sprays each of which product form employs an organic polyol antiperspirant active-containing composition of our invention.

Thus, an antiperspirant gel stick article composition of our invention employing the organic polyol antiperspirant active-containing composition of our invention contains a polyvalent metal compound-free and hydrocarbyl glycol-free, consistently-maintained stiff, clear, monophasic composition having dimensional integrity and comprises at least one fully methylated cyclic dimethyl polysiloxane having the formula [(CH₃)₂SiO]₃₋₇ serving as a lubricant, at least one C₁₂-C₁₈ monoalkanol, at least one organic C₃-C₆ alkane polyol antiperspirant active which organic polyol has bonded thereto from three to six hydroxyl moieties and/or one or more monoester, monoether, monohemiacetal, monoacetal, monohemiketal or monoketal derivatives thereof , at least one [(C₂-C₄ oxy)₅₋₁₅]C₁₂-C₁₈alkanol, at least one alkali metal C₁₂-C₂₂ alkanoate as a gellant and at least one C₂-C₃ lower alkanol solvent.

The term "consistently-maintained... composition having dimensional integrity" is intended herein to mean that when the stick article of our invention is in use in applying an organic polyol antiperspirant active-containing film to an area of occurrence of epidermal apocrine or eccrine gland sweating, the proportions of the constituents and the chemical properties of the functional composition, e.g., the fragrance composition that is evolved into the environment on use of the stick article of our invention are substantially identical to the proportions and chemical properties of the constituents originally present in the stick article and originally admixed with the remainder of the system, including the organic polyol antiperspirant active; and the geometric dimensions thereof with reference to the "x","y" and "z" axes on use thereof are substantially identical to the geometric dimensions originally existant in the stick article.

The term, "stiff" is herein intended to mean that the stick article of our invention is self-supporting and non-flowable at ambient temperatures or less and at ambient pressures, e.g., at temperatures of ≤ 35°C and at pressures of about 1 atmosphere absolute.

The term "monophasic" is herein intended to mean that the stick article of our invention on use or when not in use exists in one unitary clear phase without any phase separation resulting from the inclusion in the system of a functional composition, e.g., a fragrance composition.

Furthermore, a "roll-on" article composition of our invention employing the organic polyol antiperspirant active-composition of our invention contains a stable emulsion which comprises water as a solvent, at least one organic C₃-C₆ alkane polyol antiperspirant active which organic polyol has bonded thereto from three to six hydroxyl moieties and/or one or more monoester, monoether, monohemiacetal, monoacetal, monohemiketal or monoketal derivatives thereof, a system-compatible thickening agent which is preferably a polymer of acrylic acid cross-linked with an allyl ether of sucrose, a pH adjustment agent, preferably a tri-lower alkanolamine and most preferably triethanolamine, a preservative, preferably a hydantoin derivative and most preferably bis(hydroxymethyl)-5,5-dimethylhydantoin (1,3-bis(hydroxymethyl)-5,5-dimethylimidazolidinedione) and a lubricant/surfactant, preferably a dimethicone copolyol (a hydroxy(ethoxy)₁₅₋₃₀(propoxy)₂₋₃₀ dimethicone, where the term "dimethicone" is "a mixture of fully methylated linear siloxane polymers blocked with trimethylsiloxy units conforming to the formula:

(CH₃)₃SiO [Si(CH₃)(CH₂(C₂ and/or C₃-alkoxy)₂₋₃₀] ₂OSi(CH₃)₃").

Such compositions as are used in the practice of our invention may optionally contain, in addition, one or more fragrance components which have n-octanol/water partition coefficients, "P" such that each of the fragrance components have no molecular weight limitations and can have a C log₁₀P in the range of from about 0.5 to about 10 including, but not limited to aldehydes, ketones, esters, hemiacetals, acetals, hemiketals, ketals, alcohols, carboxylic acids, ethers, thioethers, thiols, amides, shiff bases, lactones, thiolactones, lactams, hydrocarbons, natural essential oils and nitriles without restriction concerning sensitivity of the fragrance components or combination thereof to the pH of the composition. This lack of restriction is enabled as a result of the absence of polyvalent metal compounds which, if present, can effect (i) a physical and/or visual alteration (e.g., color and/or transparency) in which the antiperspirant active is employed and (ii) a chemical alteration of the fragrance composition particularly where aldehydes such as citronellal or alkylsubstituted arylalkylcarboxaldehydes such as para-tertiary butyl-α-methylhydrocinnamaldehyde are employed. Such fragrance compositions can also be malodour-counteracting fragrance compositions as described in U.S. Patent 6,737,395 or antimicrobial fragrance compositions as described in U.S. Patent 6,495,512 or fragrance/anti-microbial compositions as described in U.S. Patent 6,517,759.

Furthermore, such compositions as are used in the practice of our invention may contain one or more anti-microbial agents such as the antimicrobial deodorant compositions as set forth in U.S. Patent Application 2003/0059396 A1 or other antimicrobial agents such as isothiazolone compounds, alkali metal pyrithiones, polymethoxy bicyclic oxazolidines and halogenated compounds as described in U.S. Patent Application 2003/0035748 A1, or halogenated phenol derivatives such as 5-chloro-2-(2,4-dichlorophenoxy)phenol.

### DETAILED DESCRIPTION OF THE INVENTION

The polyvalent metal compound-free, hydrocarbyl glycol-free composition of our invention used for topical application of the organic polyol antiperspirant active of our invention contains from about 10 to about 50 parts by weight of the organic polyol antiperspirant active; preferably from about 25 to about 40 parts by weight of the organic polyol antiperspirant active and most preferably from about 28 to about 35 parts by weight of the organic polyol antiperspirant active based on 100 parts by weight of the composition in which it is used, e.g., stick article composition, "roll-on" article composition, aerosol composition, lotion and the like. As stated above, the organic polyol antiperspirant active is at least one of of glycerine, sorbitol, sorbitol internal cyclic ether anyhydride, mannitol, mannitol internal cyclic ether anhydride having the structure: 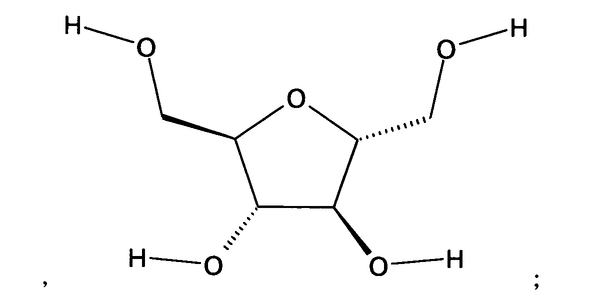 glucitol, glucitol internal cyclic ether anhydride having the structure: 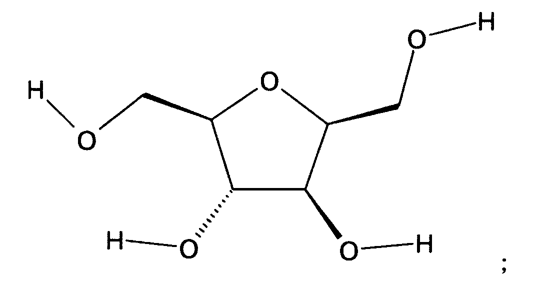 and 1,2,6-hexane triol and/or a C₂-C₁₈ alkyl or alkenyl mono-ester thereof, a C₂-C₁₂ alkyl monoether thereof, a mono[(C₂-C₃ oxy)₁₋₁₅]ether thereof, a mono-C₂-C₈ hemiacetal thereof, a mono-C₁-C₈ acetal thereof , a mono-C₃-C₈ hemiketal thereof and/or a
mono-C₃-C₈ ketal thereof. Examples of alkyl or alkenyl monoesters are glyceryl monoacetate, glyceryl/sorbitol monooleate, the mixture of glyceryl monocaprylate and glyceryl monocaprate marketed as ESTOL 3601, Uniquema Chemie, B.V. of Gouda, The Netherlands, glyceryl monopalmitate, glyceryl monoricinoleate marketed as LONZEST GMR, Lonza Inc. of Fair Lawn, New Jersey 07410 and glyceryl monoheptanoate marketed as CAPMUL 707G, Abitec Corporation of Columbus, Ohio 43215. Examples of alkyl ethers are 2-glyceryl monoisopropyl ether and 3-glyceryl-mono-n-heptyl ether. Examples of alkoxyglyceryl ethers are PPG-3-glyceryl ether (CAS No. 25791-96-2), a mixture of 1- and 2-(propoxy)₃ glyceryl ethers marketed as NEWPOL GP-250, Sanyo Chemical Co. and PPG-3-Glycereth-4, a mixture of 1- and 2-(propoxy)₃(ethoxy)₄ glyceryl monoethers. An example of an acetal useful in the practice of our invention is glycerol formal (CAS No. 5464-28-8) marketed as ELCOTAL GF, Lambiotte et Cie. An example of a ketal useful in the practice of our invention is 2,2-diglyceroxypropane.

Glycerine having the formula (H₂COH)COH(H₂COH) is the preferred organic polyol antiperspirant active for use in the practice of our invention.

The stick articles of our invention, when used in a preferred manner in the practice of our invention are contained, for example, in a package described in detail in U.S. Patents 5,275,496 and 6,386,778 and U.S. Patent Application US 2001/0002962 A.

Such packages for the stick article of our invention comprise means for supporting the organic polyol antiperspirant active-containing substance-providing stick article of our invention such that an end portion of said stick article can be exposed for use, with the means for supporting said stick article including a stick-surrounding member for surrounding the stick article, the stick-surrounding member having an opening such that the stick article can be exposed for use, and a border of the stick-surrounding member forming the opening, said border forming a top end of the supporting means, such that said end portion can be elevated from said means for supporting and can protrude therefrom so as to be exposed for use, said stick article being contained within said stick-surrounding member. Thus, for example, the means for elevating includes a screw feed mechanism having an elevator screw such that upon rotation of the elevator screw the stick article is pushed up from the bottom such that the end portion protrudes and is exposed for use. As a second example, the means for elevating is a means for pushing up the stick article so that the end portion protrudes from the means for supporting and is exposed for use.

Furthermore, our invention is directed to a process for applying a controllably releasable antiperspirant active-containing substance optionally including other functional substances such as a perfume composition or an anti-microbial composition to an area of occurrence of (i) epidermal apocrine gland sweat secretions or (ii) epidermal eccrine gland secretions comprising the steps of providing a package as described above; exposing for use the end portion of said stick article contained within said package; epidermal surface to be treated; and applying the end portion of said stick article contained within said package to a finite area of epidermal surface.

Other optional materials useful in formulation of the composition of the stick article are as follows, in the stated ranges of use:
from about 10 to about 50 parts by weight, preferably from about 25 to about 35 parts by weight of a fully methylated cyclic dimethyl polysiloxane having the formula [(CH₃)₂SiO]₃₋₇ (for example, cyclomethicone (CAS No. 69430-24-6) marketed as DOW CORNING 244 Fluid, Dow Corning Corporation of Midland, Michigan 48686-0994, and cyclotetrasiloxane (CAS No. 293-51-6) having the formula [(CH₃)₂SiO]₄ marketed as KF994, Shin-Etsu Chemical Co., Ltd. of Tokyo, Japan); from about 10 to about 30 parts by weight, preferably from about 15 to about 25 parts by weight of at least one C₁₂-C₁₈ monoalkanol (for example isostearyl alcohol (CAS No. 27458-93-1), 16-methyl-1-heptadecanol marketed as JARCOL 1-18CG, trademark of the Jarchem Co.); from about 8 parts by weight to about 15 parts by weight, preferably from about 9 to about 12 parts by weight of at least one [(C₂-C₄ oxy)₅₋₁₅]C₁₂-C₁₈ alkanyl ether (for example, PPG-10 cetyl ether ((n-propoxy)₁₀ cetane , (CAS No. 9035-85-2) marketed as PROCETYL 10, Croda, International plc of East Yorkshire, England, U.K.; from about 5 parts by weight to about 15 parts by weight, preferably from about 7 to about 11 parts by weight of at least one alkali metal C₁₂-C₂₂ alkanoate (for example, and preferably sodium stearate); and from about 5 parts by weight to about 25 parts by weight, preferably from about 6 to about 10 parts by weight, of at least one C₂-C₃ lower alkanol (for example cosmetic-grade ethanol or isopropanol).

When an optional antibacterial agent is employed in the formulation, for example, 5-chloro-2-(2,4-dichlorophenoxy)phenol, the range of the amount used in the formulation is from about 0.001 to about 0.5 parts by weight, preferably from about 0.08 to about 0.3 parts by weight.

Similarly, a "roll-on" article employing an antiperspirant "roll-on" composition containing the organic polyol antiperspirant active of our invention may be used for topical application of a "roll-on" antiperspirant composition to the area of occurrence of (i) epidermal apocrine gland sweat secretions or (ii) epidermal eccrine gland secretions of the organic polyol antiperspirant active-containing composition in accordance with the practice of our invention. Such "roll-on" articles useful in the practice of our invention are described in U.S. Patent 5,553,957.

Other optional materials useful in formulation of the composition useful in conjunction with the "roll-on" article are:
from about 50 to about 70 parts by weight, preferably from about 55 to about 65 parts by weight of water;
from about 0.2 to about 0.5 parts by weight, preferably about 0.3 parts by weight of a at least one polymer of acrylic acid cross-linked with the allyl ether of sucrose (CAS No. 9003-01-4) for example Carbomer 934, marketed as CARBOPOL 934, Noveon IP Holdings Corp. of Cleveland, Ohio 44141-3247.;
from about 0.7 to about 1.4 parts by weight, preferably about 1.0 parts by weight of triethanolamine;
from about 0.001 to about 0.5 parts by weight, preferably about 0.3 parts by weight of DMDM hydantoin (CAS No. 6440-58-0)(bis(hydroxymethyl)-5,5-dimethylhydantoin marketed as GLYDANT, Lonza, Inc. of Fair Lawn, New Jersey 07410;
from about 1.75 to about 3.0 parts by weight, preferably about 2.3 parts by weight of dimethicone polyol for example SILSOFT 430 dimethicone polyol and SILSOFT 475 dimethicone polyol, Crompton Corporation of Greenwich, Connecticut 06831 and DOW CORNING 193 surfactant, Dow Corning Corporation of Midland, Michigan 48686-0994.

Similarly, an "aerosol" device employing an antiperspirant composition containing the organic polyol antiperspirant active of our invention admixed with a propellant such as 1,1,1,2,2-pentafluorobutane may be used for topical application of an aerosol antiperspirant composition to the area of occurrence of (i) epidermal apocrine gland sweat secretions or (ii) epidermal eccrine gland secretions of the organic polyol antiperspirant active-containing composition in accordance with the practice of our invention. Such "aerosol" devices useful in the practice of our invention are described at Column 23, lines 48-67 and Column 24, lines 1-11 and lines 25-30 of U.S. Patent 6,682,694.

When an optional fragrance component or composition is employed in the stick article formulation or in a "roll-on" article formulation, or with an "aerosol" device, for example, a fragrance composition containing the components as set forth in the following Table II:

**TABLE II**

| *Fragrance Component* | Clog₁₀P value | Molecular Weight | Parts by Weight |
|---|---|---|---|
| 1-phenyl hexanol-5 | 3.299 | 178.28 | 3.0 |
| α-santalol | 3.800 | 220.36 | 5.0 |
| iso-eugenol | 2.547 | 164.21 | 8.0 |
| amyl salicylate | 4.601 | 208.26 | 4.0 |
| benzyl salicylate | 4.383 | 228.25 | 9.0 |
| β-caryophyllene | 6.333 | 204.36 | 3.0 |
| cedrol | 4.530 | 222.37 | 5.0 |
| cedryl acetate | 5.436 | 264.41 | 5.0 |
| cedryl formate | 5.070 | 238.37 | 1.5 |
| cyclohexyl salicylate | 5.265 | 220.29 | 9.0 |
| γ-dodecalactone | 4.359 | 198.31 | 7.5 |
| ethyl undecylenate | 4.888 | 212.34 | 7.7 |
| geranyl anthranilate | 4.216 | 273.38 | 7.7 |
| β-phenylethyl benzoate | 4.058 | 226.38 | 3.0 |
| β-phenylethyl phenyl acetate | 3.767 | 240.31 | 3.5 |
| 5-acetyl-1,1,2,3,3,6-hexamethyl indane | 5.977 | 258.41 | 5.6 |
| cyclopentadecanolide | 6.246 | 240.39 | 7.3 |
| d-limonene | 4.232 | 136.24 | 2.0 |
| cis-p-t-butylcyclohexyl acetate | 4.019 | 198.31 | 1.5 |
| amyl cinnamic aldehyde | 4.324 | 202.30 | 5.5 |
| linalyl benzoate | 5.233 | 258.36 | 4.4 |

the range of use of the fragrance component or fragrance composition is in the range of from about 0.01 parts to about 5.0 parts by weight, preferably from abou 0.02 to about 2 parts by weight.

The following examples are not meant to define or otherwise limit the scope of the invention. Rather the scope of the invention is to be ascertained according to the claims that follow the examples.

### EXAMPLE I

A clear antiperspirant stick was prepared as follows:

The following materials were admixed:

| Ingredients | Parts by Weight |
|---|---|
| Cyclomethicone (TBF-505 Silicone Fluid marketed by Path Silicone Co.) | 39.70 |
| Isostearyl Alcohol (JARCOL 1-18CG marketed by the Jarchem Co.) | 19.80 |
| Glycerine | 18.90 |
| (Propoxy)₁₀Cetyl Ether (PROCETYL 10 marketed by Croda International plc of East Yorkshire, England, U.K. | 10.00 |
| Sodium Stearate | 8.00 |
| 5-chloro-2-(2,4-dichlorophenoxy)phenol(IRGASAN DB-300, marketed by Ciba Specialty Chemicals Corporation of Tarrytown, New York 10591) | 0.10 |

The resulting mixture was heated until clear (90°C for a period of 0.3 hours). The clear mixture was cooled to 45°C and admixed with 7.50 parts by weight of ethanol (alcohol SD#40) the resulting molten, clear mixture was poured into a mold, whereby an antiperspration stick was formed. The antiperspirant stick was then placed in an article as described in U.S. Patent 5,275,496.

The antiperspirant stick was used and compared with a stick where the glycerine was replaced with a mixture of aluminum chlorhydroxide, zirconyl hydroxychloride and glycine in a ratio of 7.5:4.6:2.0 and the sodium stearate gelling agent was replaced with a dibenzylidene sorbitol gelling agent. Each of the sticks had an equivalent antiperspirant efficacy with respect to (i) degree of reduction of epidermal aprocrine gland perspiration; (ii) degree of reduction of epidermal eccrine gland perspiration; and (iii) time of effectiveness. A 10 member panel unanimously preferred the stick containing the glycerine in view of the total lack of irritancy on use thereof.

### EXAMPLE II

A "roll-on" antiperspirant emulsion for use with the "roll-on" article illustrated in Figure 1 and 4 of U.S. Patent 5,553,957 was prepared as follows:

The following materials were admixed in the manner set forth below:

| Ingredients | Parts by Weight |
|---|---|
| Deionized water | 61.9 |
| Carbomer 934 (polymer of acrylic acid cross linked with the allyl ether of sucrose) marketed as CARBOPOL 934, Noveon IP Holdings Corp.) | 0.3 |
| triethanolamine | 1.0 |
| glycerine | 34.2 |
| DMDM Hydantoin (bis(hydroxymethyl)-5,5-dimethylhydantoin) marketed as GLYDANT, Lonza, Inc. | 0.3 |
| Dimethicone Polyol (DOW CORNING 193 Surfactant) | 2.3 |

The CARBOPOL was dispersed in the water, and subjected to intense agitation until a single liquid phase (solution) existed. The triethanolamine was then added to the resulting CARBOPOL solution, with stirring. The remaining ingredients were added to the resulting solution. The resulting product was employed in the "roll-on" article of U.S. Patent 5,553,957, yielding excellent results as an antiperspirant.

Each of the disclosures of each of the U.S. Patents and Published U.S. Patent Applications cited herein is herein incorporated by reference. The reader is directed to read the patent applications referred to now and to consider their disclosure as part of the disclosure of this patent application.

Optional features disclosed herein, including optional features of the claims (e.g. dependent claims) may be used together in any combination and in any number.

## Claims

1. A method for inhibiting apocrine gland sweating or eccrine gland sweating comprising the step of topically applying to at least one area of occurrence of (i) epidermal apocrine gland sweat secretions or (ii) epidermal eccrine gland secretions, an organic polyol antiperspirant active-containing composition comprising a sweat secretion inhibiting concentration of a C₃-C₆ alkane polyol antiperspirant active which polyol has bonded thereto from three to six hydroxyl moieties and/or monoester, monoether,monohemiacetal, monoacetal, monohemiketal or monoketal derivatives thereof and is selected from the group consisting of glycerine, sorbitol, sorbitol internal cyclic ether anyhydride, mannitol, mannitol internal cyclic ether anhydride, glucitol, glucitol internal cyclic ether anhydride and 1,2,6-hexane triol and a C₂-C₁₈ alkyl or alkenyl mono-ester thereof, a C₂-C₁₂ alkyl monoether thereof, a mono[(C₂-C₃ oxy)₁₋₁₅]ether thereof, a mono-C₂-C₈ hemiacetal thereof, a mono-C₁-C₈ acetal thereof, a mono-C₃-C₈ hemiketal thereof and a mono-C₃-C₈ ketal thereof in the absence of (i) polyvalent metal compounds or (ii) glycols of the formula R(OH)₂ where R is hydrocarbyl.

2. A method for inhibiting apocrine gland sweating or eccrine gland sweating comprising the step of topically applying to at least one area of occurrence of (i) epidermal apocrine gland sweat secretions or (ii) epidermal eccrine gland secretions, a sweat secretion inhibiting amount of a glycerine antiperspirant active in the absence of (i) polyvalent metal compounds and (ii) glycols of the formula R(OH)₂ where R is hydrocarbyl.

3. The method of claim 1 wherein the organic polyol antiperspirant active-containing composition is in the form of a stick article having a consistently-maintained stiff, clear, monophasic composition having dimensional integrity and comprises at least one fully methylated cyclic dimethyl polysiloxane having the formula [(CH₃)₂SiO]₃₋₇, at least one C₁₂-C₁₈ monoalkanol, glycerine, at least one [(C₂-C₄ oxy)₅₋₁₅]C₁₂-C₁₈ alkanol, at least one alkali metal C₁₂-C₂₂ alkanoate and at least one C₂-C₃ lower alkanol.

4. The method of claim 3 wherein the organic polyol antiperspirant active-containing composition additionally comprises an antimicrobial substance.

5. The method of claim 3 wherein the organic polyol antiperspirant active-containing composition additionally comprises a fragrance.

6. The method of claim 3 wherein the organic polyol antiperspirant active-containing composition comprises:
(a) from about 10 to about 50 parts by weight of a fully methylated cyclic dimethyl polysiloxane having the formula [(CH₃)₂SiO]₃₋₇;
(b) from about 10 to about 30 parts by weight of at least one C₁₂-C₁₈ monoalkanol;
(c) from about 10 to about 50 parts by weight of glycerine antiperspirant active;
(d) from about 8 parts by weight to about 15 parts by weight of at least one
[(C₂-C₄ oxy)₅₋₁₅]C₁₂-C₁₈ alkanyl ether;
(e) from about 5 parts by weight to about 15 parts by weight of at least one alkali metal C₁₂-C₂₂ alkanoate; and
(f) from about 5 parts by weight to about 25 parts by weight of at least one C₂-C₃ lower alkanol.

7. The method of claim 6 wherein the organic polyol antiperspirant active-containing composition additionally comprises from about 0.001to about 0.50 parts by weight of an antimicrobial substance.

8. The method of claim 6 wherein the organic polyol antiperspirant active-containing composition additionally comprises from about 0.01 to about 5.0 parts by weight of a compatible fragrance and, optionally, from about 0.001 to about 0.50 parts by weight of an antimicrobial substance.

9. The method of claim 7 wherein the antimicrobial substance is 5-chloro-2-(2,4-dichlorophenoxy)phenol.

10. The method of claim 8 wherein the compatible fragrance comprises at least one malodour-counteracting fragrance component and/or at least one antimicrobial fragrance component.

11. An antiperspirant stick article having a consistently-maintained stiff, clear, monophasic composition having dimensional integrity comprising at least one fully methylated cyclic dimethyl polysiloxane having the formula [(CH₃)₂SiO]₃₋₇, at least one C₁₂-C₁₈ monoalkanol, at least one [(C₂-C₄ oxy)₅₋₁₅]C₁₂-C₁₈ alkanol, at least one alkali metal C₁₂-C₂₂ alkanoate, at least one C₂-C₃ lower alkanol and at least one C₃-C₆ alkane polyol antiperspirant active which polyol has bonded thereto from three to six hydroxyl moieties and/or monoester, monoether, monohemiacetal, monoacetal, monohemiketal or monoketal derivatives thereof and is selected from the group consisting of glycerine, sorbitol, sorbitol internal cyclic ether anyhydride, mannitol, mannitol internal cyclic ether anhydride, glucitol, glucitol internal cyclic ether anhydride, 1,2,6-hexane triol, a C₂-C₁₈ alkyl or alkenyl mono-ester thereof, a C₂-C₁₂ alkyl monoether thereof, a mono[(C₂-C₃ oxy)₁₋₁₅]ether thereof, a mono-C₂-C₈ hemiacetal thereof, a mono-C₁-C₈ acetal thereof, a mono-C₃-C₈ hemiketal thereof and a mono-C₃-C₈ ketal thereof in the absence of (i) any polyvalent metal compounds or (ii) glycols of the formula R(OH)₂ where R is hydrocarbyl.

12. A package for utilization of the polyol antiperspirant active-containing composition-providing stick article of claim 11 comprising means for supporting said polyol antiperspirant active-containing composition-providing stick article such that an end portion of said stick article can be exposed for use, the means for supporting said stick article including a stick-surrounding member for surrounding the stick article, the stick-surrounding member having an opening such that the stick article can be exposed for use, and a border of the stick-surrounding member forming the opening, said border forming a top end of the supporting means, such that said end portion can be elevated from said means for supporting and can protrude therefrom so as to be exposed for use, said stick article being contained within said stick-surrounding member.

13. The package of claim 12 wherein said means for elevating includes a screw feed mechanism having an elevator screw such that upon rotation of the elevator screw the stick article is pushed up from the bottom such that the end portion protrudes and is exposed for use.

14. The package of claim 12 wherein the means for elevating is a means for pushing up the stick article so that the end portion protrudes from the means for supporting and is exposed for use.

15. A process for applying a controllably releasable polyol antiperspirant active-containing antiperspirant composition to a mammalian epidermal surface area of occurrence of (i) epidermal apocrine gland sweat secretions or (ii) epidermal eccrine gland secretions comprising the steps of providing the package of claim 12; exposing for use the end portion of said stick article contained within said package; providing a mammalian epidermal surface to be treated; and applying the end portion of said stick article contained within said package to a finite area of said mammalian epidermal surface.

16. The method of claim 6 wherein the polyol antiperspirant active-containing composition comprises (a) from about 10 to about 50 parts by weight of a fully methylated cyclic dimethyl polysiloxane having the formula [(CH₃)₂SiO]₃₋₇; (b) from about 10 to about 30 parts by weight of isostearyl alcohol; (c) from about 10 to about 50 parts by weight of glycerine antiperspirant active; (d) from about 8 to about 15 parts by weight of decapropoxy-n-cetane; (e) from about 5 to about 15 parts by weight of sodium stearate; (f) from about 5 to about 25 parts by weight of ethanol and (g) from about 0.001 to about 0.5 parts by weight of 5-chloro-2-(2,4-dichlorophenoxy)phenol.

17. The antiperspirant stick article of claim 11 comprising (a) from about 10 to about 50 parts by weight of a fully methylated cyclic dimethyl polysiloxane having the formula [(CH₃)₂SiO]₃₋₇; (b) from about 10 to about 30 parts by weight of isostearyl alcohol; (c) from about 10 to about 50 parts by weight of glycerine antiperspirant active; (d) from about 8 to about 15 parts by weight of decapropoxy-n-cetane; (e) from about 5 to about 15 parts by weight of sodium stearate; (f) from about 5 to about 25 parts by weight of ethanol and (g) from about 0.001 to about 0.5 parts by weight of 5-chloro-2-(2,4-dichlorophenoxy)phenol.

18. The method of claim 16 wherein the organic polyol antiperspirant active-containing composition additionally comprises from about 0.01 to about 5.0 parts by weight of a fragrance.

19. The antiperspirant stick article of claim 17 which additionally comprises from about 0.01 to about 5.0 parts by weight of a fragrance.

20. The antiperspirant stick article of claim 19 wherein the fragrance comprises at least one malodour-counteracting fragrance component and/or at least one antibacterial fragrance component.

21. The method of claim 1 wherein the topical application is effected by means of a roll- on or by means of an aerosol spray.
